# EUROPEAN PATENT APPLICATION

(11) **EP 4 588 820 A1**
(43) Date of publication of application: **23.07.2025**
(21) Application number: 23864958.6
(22) Date of filing: 05.04.2023
(51) Int. Cl.: B65D 75/62, B65D 85/07

(54) **ABSORBENT ARTICLE PACKAGING POUCH AND ABSORBENT ARTICLE PACKAGING PRODUCT**

(30) Priority: 15.09.2022 JP 2022146676
(71) Applicant: DAIO PAPER CORPORATION, Shikokuchuo-shi Ehime 799-0492 (JP)
(72) Inventor: KURIHARA, Ryoko, Sakura-shi, Tochigi 329-1411 (JP)
(74) Representative: Epping - Hermann - Fischer
(86) International application number: PCT/JP2023/014030
(87) International publication number: WO 2024/057592

(57) **Abstract**

An absorbent article packaging bag that is colored and capable of accommodating a plurality of absorbent articles in an upright state, and the absorbent article packaging bag includes a substantially rectangular parallelepiped shape having a top surface portion, a bottom surface portion, and a surrounding surface portion connecting the top surface portion and the bottom surface portion. The surrounding surface portion has an inside visible region, and part of the absorbent articles is visible through the inside visible region. In the surrounding surface portion, an easy-to-tear portion with which the surrounding surface portion can be torn in a top-bottom direction is formed, and the easy-to-tear portion at least partially overlaps the inside visible region.

## Description

### TECHNICAL FIELD

The present invention relates to an absorbent article packaging bag and an absorbent article packaged product.

### BACKGROUND ART

In general, absorbent articles such as incontinence pads, sanitary napkins, and disposable diapers are provided in a state in which the absorbent articles are individually folded and packaged to form individual packages, and a plurality of such individual packages are accommodated in a packaging bag.

Many of the packaging bags as described above are provided with a linear portion (easy-to-tear portion) that can be easily torn, such as a perforation, and are configured to be able to form an opening for taking out the individual package by tearing the packaging bag at the linear portion. For example, Patent Document 1 describes a packaging bag for paper diapers or the like, in which a tear line is provided substantially parallel to the bottom surface of the packaging bag in a range extending from a position below the seal line of the upper opening to the upper surface of the packaged article to be filled and accommodated or to the vicinity thereof.

### RELATED ART DOCUMENTS

### PATENT DOCUMENT

Patent Document 1: Japanese Unexamined Patent Publication No. 2001-301859

### SUMMARY OF THE INVENTION

### PROBLEMS TO BE SOLVED BY THE INVENTION

An easy-to-tear portion formed in a packaging bag is often difficult to visually recognize and find. Therefore, it takes time and effort for a user to find the easy-to-tear portion to open the packaging bag, and the user may feel stress.

In view of the above, an object of one aspect of the present invention is to provide a configuration in which an easy-to-tear portion in an absorbent article packaging bag is easily found and which does not require time and effort to open the packaging bag.

### MEANS FOR SOLVING THE PROBLEMS

An aspect according to the present invention is a colored absorbent article packaging bag that can accommodate a plurality of absorbent articles in an upright state, the packaging bag having a substantially rectangular parallelepiped shape having a top surface portion, a bottom surface portion, and a surrounding surface portion between the top surface portion and the bottom surface portion, the surrounding surface portion having an inside visible region through which part of the absorbent articles is visible, the surrounding surface portion having an easy-to-tear portion with which the surrounding surface portion can be torn in a top-bottom direction, and the easy-to-tear portion at least partially overlapping the inside visible region.

### EFFECTS OF THE INVENTION

According to one aspect of the present invention, it is possible to provide a configuration in which an easy-to-tear portion in an absorbent article packaging bag is easily found and it does not take time and effort to open the packaging bag.

### BRIEF DESCRIPTION OF THE DRAWINGS

[FIG. 1] FIG. 1 is a perspective view of an absorbent article packaged product in which absorbent articles are accommodated in an absorbent article packaging bag according to a first embodiment.
[FIG. 2] FIG. 2 is an unfolded-open view of the absorbent article packaging bag of FIG. 1.
[FIG. 3] FIG. 3 is a diagram for explaining a use of the absorbent article packaged product illustrated in FIG. 1.
[FIG. 4] FIG. 4 is a perspective view of an absorbent article packaged product according to a modified example of the first embodiment.
[FIG. 5] FIG. 5 is an unfolded-open view of the absorbent article packaging bag of FIG. 4.
[FIG. 6] FIG. 6 is a perspective view of an absorbent article packaged product according to another modified example of the first embodiment.
[FIG. 7] FIG. 7 is an unfolded-open view of the absorbent article packaging bag of FIG. 6.
[FIG. 8] FIG. 8 is a perspective view of an absorbent article packaged product according to still another modified example of the first embodiment.
[FIG. 9] FIG. 9 is an unfolded-open view of the absorbent article packaging bag of FIG. 8.
[FIG. 10] FIG. 10 is a diagram for explaining an advantage of the absorbent article packaged product illustrated in FIGS. 8 and 9.
[FIG. 11] FIG. 11 is a perspective view of an absorbent article packaged product according to still another modified example of the first embodiment.
[FIG. 12] FIG. 12 is an unfolded-open view of the absorbent article packaging bag of FIG. 11.
[FIG. 13] FIG. 13 is a perspective view of an absorbent article packaged product according to still another modified example of the first embodiment.
[FIG. 14] FIG. 14 is a perspective view of an absorbent article packaged product in which absorbent articles are accommodated in an absorbent article packaging bag according to a second embodiment.
[FIG. 15] FIG. 15 is an unfolded-open view of the absorbent article packaging bag of FIG. 14.
[FIG. 16] FIG. 16 is a perspective view of an absorbent article packaged product according to a modified example of the second embodiment.
[FIG. 17] FIG. 17 is an unfolded-open view of the absorbent article packaging bag of FIG. 16.
[FIG. 18] FIG. 18 is a perspective view of an absorbent article packaged product according to another modified example of the second embodiment.
[FIG. 19] FIG. 19 is an unfolded-open view of the absorbent article packaging bag of FIG. 19.
[FIG. 20] FIG. 20 is a perspective view of an absorbent article packaged product according to still another modified example of the second embodiment.

### DESCRIPTION OF EMBODIMENTS

Hereinafter, embodiments of the present invention will be described with reference to the drawings. In the drawings, the same or corresponding components are denoted by the same reference numerals unless otherwise specified, and the description thereof may be omitted.

### <First Embodiment>

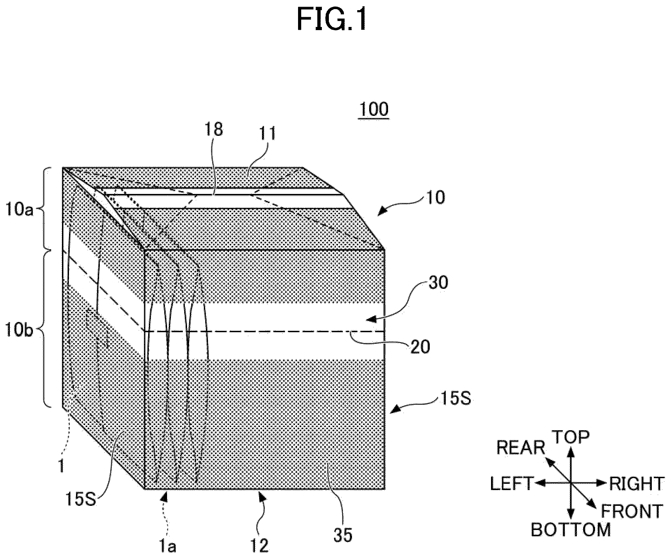

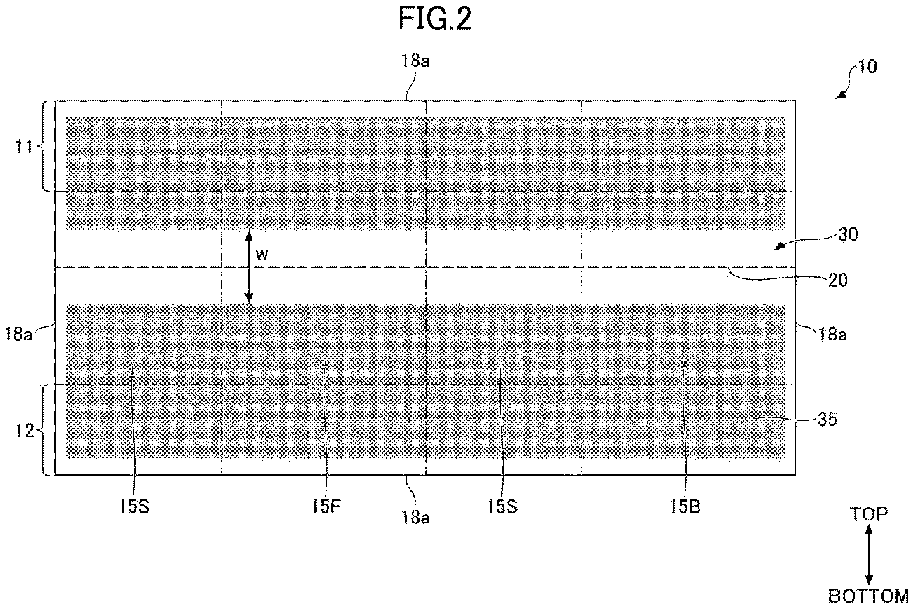

FIG. 1 illustrates an absorbent article packaged product 100 including an absorbent article packaging bag (hereinafter, also simply referred to as a "packaging bag") 10 according to the first embodiment and a plurality of absorbent articles 1 accommodated in the absorbent article packaging bag 10. FIG. 2 is an unfolded-open view of the absorbent article packaging bag 10 illustrated in FIG. 1. The absorbent article 1 may be an incontinence pad, a sanitary napkin, a pantyliner, a disposable diaper, or the like, and may have a configuration including a liquid permeable top sheet, a liquid impermeable back sheet, and an absorbent body disposed between these sheets. In the example illustrated in the drawings, the absorbent article 1 is an incontinence pad.

As illustrated in FIG. 1, the plurality of absorbent articles 1, 1, ... are arranged in an upright state and stacked in the thickness-wise direction of the absorbent articles 1 to form an absorbent article row 1a. In the first embodiment, one absorbent article row 1a is accommodated in the packaging bag 10. In this specification, the direction in which the absorbent articles 1 are upright will be referred to as the "top-bottom direction", the direction in which the absorbent articles 1 are arranged will be referred to as the "left-right direction" (lateral direction), and the direction orthogonal to the top-bottom direction and the left-right direction will be referred to as the "front-rear direction".

In the example of FIG. 1, each absorbent article 1 is in a state of being folded and packaged by a packaging sheet (an individual package). The individual package of the absorbent article 1 may have dimensions of 50 to 200 mm in the longitudinal direction, 50 to 200 mm in the lateral direction, and 3 to 30 mm in the thickness direction. The absorbent article 1 does not need to be packaged individually. In other words, in the present specification, the absorbent article 1 may be an absorbent article either not individually packaged or individually packaged (an individual package of the absorbent article).

### (Packaging Bag)

As illustrated in FIG. 1, the packaging bag 10 has a substantially rectangular parallelepiped shape as a whole. In the present specification, the "substantially rectangular parallelepiped shape" is a polyhedron having six rectangular (including square) surfaces. The substantially rectangular parallelepiped shape does not necessarily have clear edges and/or vertices, and the edges and/or vertices may be rounded. In other words, the shape may be a three-dimensional shape having six substantially rectangular surfaces. The apex angle of the "substantially rectangular" surface may be approximately a right angle, and may deviate from a right angle by preferably ±10°, more preferably ±5°. The packaging bag 10 having such a substantially rectangular parallelepiped shape has a top surface portion 11 on the upper side, a bottom surface portion 12 on the lower side, and a surrounding surface portion 15 between the top surface portion 11 and the bottom surface portion 12. The surrounding surface portion 15 has a front surface portion 15F on the front side, a rear surface portion 15B on the rear side, and left and right side surface portions 15S and 15S. The front surface portion 15F is a surface that faces consumers when the packaging bags 10 are placed in a store or the like by a normal way, and article-related information (described later in detail) is displayed most conspicuously on the front surface portion 15F among the surfaces included in the packaging bag 10.

The size of the packaging bag 10 according to the first embodiment (in the case of accommodating one absorbent article row) may be about 80 to 300 mm in the left-right direction, 60 to 200 mm in the front-rear direction, and 70 to 150 mm in the top-bottom direction.

The packaging bag 10 is formed of a sheet material. The type of the sheet material is not limited to a particular type, and may be a resin, non-woven fabric, paper, or the like. In the case where the type of the sheet material is a resin, the sheet material may be a resin film such as a polyethylene film or a polypropylene film. The packaging bag 10 may be a single sheet obtained by laminating or joining two or more layers of the same or different types of sheet materials, for example, a laminate film formed of paper and a resin film. The packaging bag 10 may be formed by layering two or more sheets of the same or different kinds of sheet materials without joining them.

A packaging method using the packaging bag 10 is not limited to a particular method, but is preferably gusset packaging as illustrated in FIG. **1****.** The packaging bag 10 can be created by, for example, forming a long tubular sheet material by joining both edges of a long sheet material, forming a gusset by folding both ends of the long tubular sheet material in the width direction orthogonal to the longitudinal direction, and joining the sheet material in the thickness direction along the width direction at a predetermined position in the longitudinal direction (at a position between absorbent article packaged products). To join the sheet materials, heat sealing, ultrasonic sealing, an adhesive, or the like can be used. Therefore, the joined portion is formed in each of the top surface portion 11 and the bottom surface portion 12 of the packaging bag 10, but in FIG. 1, only the joined portion 18 formed in the top surface portion 11 is illustrated, and illustration of the joined portion on the bottom surface portion 12 is omitted. The configuration of the gusset in the bottom surface portion 12 is also omitted in the drawings. FIG. 2 illustrates a to-be-joined portion 18a that is slated to be the joined portion 18.

As illustrated in FIG. 2, the to-be-joined portion 18a is also formed between one side surface portion 15S and the rear surface portion 15B in each of the top surface portion 11 and the bottom surface portion 12. As illustrated in FIGS. 1 and 2, the end edge of the to-be-joined portion 18a does not need to be colored. The end edge slated to be the to-be-joined portion 18a corresponds to a cut position of the packaging bag 10 when the sheet material to be the packaging bag 10 is cut out or after the absorbent articles 1 are accommodated, and therefore, the end edge can be a mark of the cut position because it is not colored. Therefore, the entire region of the packaging bag 10 except an inside visible region 30 does not need to be the colored region 35, and there may be a region that is not colored in the region other than the inside visible region 30. Further, since the joined portion 18 is a portion formed by joining the planes of the sheet materials to each other, it is possible to prevent a decrease in joint strength by not coloring the joined portion 18 with ink or the like. Therefore, the case in which the joined portion 18 is not colored is particularly suitable for a case in which the joined portion 18 has a width, which is recognized as a so-called ear.

The packaging bag 10 is colored. The coloring is preferably applied to at least the front surface portion 15F, and more preferably to three surfaces, namely the front surface portion 15F and the side surface portions 15S and 15S. It is preferable that the entire surrounding surface portion 15 of the packaging bag 10, or the entire surfaces of the packaging bag 10, be colored (FIG. 2). The region (colored region) 35 where the packaging bag 10 is colored is an opaque or translucent region, and is formed so that the inside cannot be viewed through the colored region 35.

The color of the colored region 35 is not limited to a particular color but is preferably a color recognized as beige, brown, black, gray, or the like. Furthermore, the chroma in the Munsell color system may be preferably 8 or less, and more preferably 6 or less. Since these colors are not so conspicuous in a normal indoor environment, the packaging bag in these colors is not readily recognized as a packaging bag for absorbent articles. The colored region 35 may be a region excluding the inside visible region 30 (described later).

The coloring (creating the colored region 35) may be performed by printing on the sheet material constituting the packaging bag 10, or may be performed by adding a coloring agent to the material of the sheet material at the stage of manufacturing the sheet material. In the case of a sheet formed by laminating two or more sheet materials, one of the sheet materials from among the outer side-sheet material and the inner side-sheet material may be transparent, and the other may be a colored sheet material having a partially removed region. The partially removed region is removed so as to correspond to an inside visible region (described in detail later).

The colored region 35 is provided in a range such that the accommodated absorbent articles 1 are not viewed, at least when viewed from the front, over the entirety of at least one of the longitudinal direction, the lateral direction, and the thickness direction. In other words, the colored region 35 is provided in a range and a size such that the absorbent articles 1 are not viewed, at least when viewed from the front, from one end to the other end in the longitudinal direction and/or from one end to the other end in the lateral direction and from one end to the other end in the thickness direction. For example, the colored region of the packaging bag 10 at least in the front surface portion 15F can be preferably 60% or more, more preferably 70% or more, of the entire area of the front surface portion 15F. In the packaging bag 10 as a whole, the colored region of the packaging bag 10 can be preferably 60% or more, more preferably 70% or more, of a total area of the outer surfaces of the packaging bag 10 (the total area of the surfaces of the substantially rectangular parallelepiped). The colored region 35 can prevent the contents of the absorbent article packaged product 100 from being immediately recognizable at a glance. This can reduce the embarrassment or the like evoked when most of the appearance of the absorbent articles 1 is visible through the absorbent article packaging bag 10.

In this way, the colored packaging bag 10 makes the inside of the packaging bag 10 not visible and can reduce the embarrassment caused by the absorbent articles 1 being conspicuous. However, if the entire packaging bag 10 is the colored region 35 and the contents cannot be seen at all, some users feel inconvenience or anxiety. For example, some users want to avoid the contents of the packaging bag 10 being recognized at a glance, but want to check whether or not the absorbent articles are actually accommodated, or want to partially check the appearance (thickness, color, etc.) of the contained absorbent articles.

In contrast, in the packaging bag 10 according to the present embodiment, as illustrated in FIGS. 1 and 2, the inside visible region 30 is formed at least in the surrounding surface portion 15. The inside visible region 30 is a region through which the contents of the packaging bag 10 can be viewed. Thus, the inside visible region 30 may be transparent or translucent. The inside visible region 30 may be lightly colored or may include a small pattern as long as the inside visible region 30 has a transmittance that allows the presence of the accommodated absorbent articles 1 to be recognized when the inside of the packaging bag 10 is viewed through the inside visible region 30 in a normal way. The inside visible region 30 makes it easy to check whether or not the absorbent articles are actually accommodated, whether or not the absorbent articles are partially visible, the quantity of the absorbent articles remaining after the packaging bag 10 is opened, and the like, while preventing the entire appearance of the absorbent articles 1 from being viewed from the outside of the packaging bag 10. This can reduce anxiety or inconvenience that users may feel, as compared to a case where the entire packaging bag 10 is colored.

The inside visible region 30 is formed in the surrounding surface portion 15. The inside visible region 30 is preferably formed at least on the front surface portion 15F, which is easily visible or which is usually first viewed by users. As illustrated in FIGS. 1 and 2, the inside visible region 30 may be formed in an annular shape so as to surround the packaging bag 10 over the entire surrounding surface portion 15. This is preferable in that the absorbent articles 1 can be partially viewed not only from the front but also from the other surrounding surfaces.

The shape of the inside visible region 30 is preferably a shape elongated in the left-right direction (lateral direction). Thus, even in the case where the inside visible region 30 has a small area, users can view the absorbent article row 1a of the absorbent articles 1, 1, ... arranged (aligned) in the left-right direction across the thickness direction of the absorbent article **1;** therefore, users can easily check the number of the absorbent articles 1, 1, ... and the thickness of each absorbent article **1.** The elongated shape of the inside visible region 30 is also preferable from the viewpoint of the relationship with the easy-to-tear portion 20, which is described later. In the case where the inside visible region 30 has an elongated shape, the inside visible region 30 may have a width w (the dimension in the top-bottom direction; see FIG. 2) of about 10 to 30 **mm.** The size of the inside visible region 30 may be a size that can accommodate a circle with a radius of 10 mm, for example.

Furthermore, the area of the inside visible region 30 on one surface of the substantially rectangular parallelepiped shape may be preferably 2 to 40% or more, and more preferably 3 to 30% of the entire area of the one surface. With regard to the total area of the packaging bag 10, the total area of the inside visible region 30 may be preferably 2 to 40%, more preferably 3 to 30%, of the total outer surface area of the packaging bag 10 (the total area of the surfaces of the substantially rectangular parallelepiped).

### (Easy-to-tear Portion)

Furthermore, as illustrated in FIGS. 1 and **2****,** the packaging bag 10 according to the present embodiment is formed with an easy-to-tear portion 20 with which the packaging bag 10 can be torn open in the top-bottom direction. The expression "can be torn open in the top-bottom direction" means that the packaging bag 10 divided by tearing the easy-to-tear portion 20 is separated in the top-bottom direction into sections. Therefore, the easy-to-tear portion 20 extends along a direction substantially orthogonal to the top-bottom direction. The expression "substantially orthogonal" may be not only completely orthogonal to the top-bottom direction, but may include the range of preferably ±15°, more preferably ±10°, with respect to the orthogonal direction.

The easy-to-tear portion 20 is a linear portion where the packaging bag 10 is weakened. For example, the easy-to-tear portion 20 may be a perforation as illustrated in FIGS. 1 and 2. A perforation is a linear portion consisting of cut portions, which are cuts made in the sheet material constituting the packaging bag 10, and tie portions, in which no cuts are made, are alternately continuous. The easy-to-tear portion 20 is not limited to a perforation, and in order to form the easy-to-tear portion 20 the sheet material may be weakened by reducing the thickness of the sheet material, differentiating the rigidity of the sheet material, modifying the material of the sheet material, or the like.

In the example illustrated in FIGS. 1 and 2, the easy-to-tear portion 20 is formed in an annular shape so as to surround the packaging bag 10 in a circumferential direction around the top-bottom direction as an axis. In other words, the easy-to-tear portion 20 is continuously formed over the surrounding surface portion 15, more specifically, over the front surface portion 15F, the rear surface portion 15B, the two side surface portions 15S and 15S. Therefore, in the case where the easy-to-tear portion 20 is entirely torn, the packaging bag 10 can be separated into an upper section and a lower section.

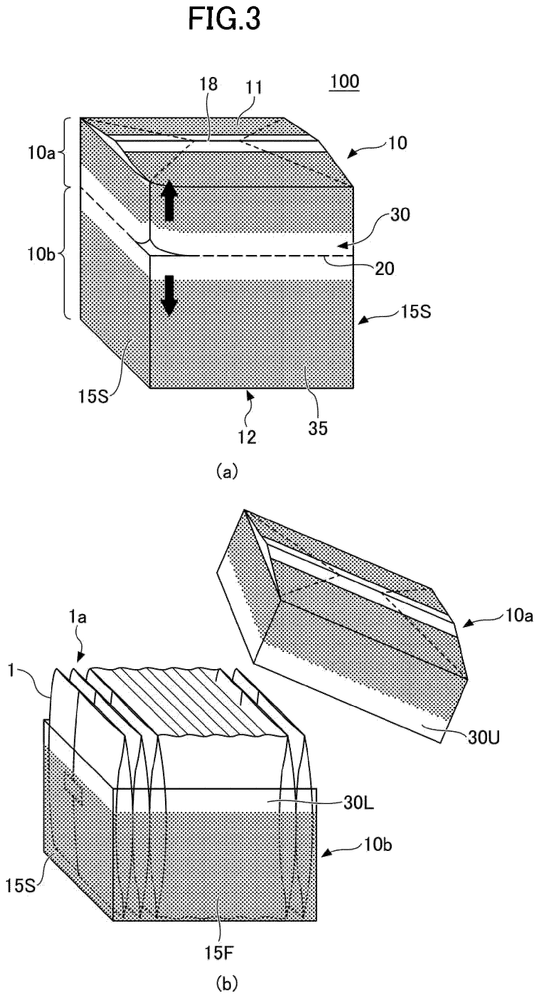

The tearing of the packaging bag 10 at the easy-to-tear portion 20 will be further described with reference to FIG. 3. As illustrated in FIG. 3(a), for example, a user can take the upper portion of the easy-to-tear portion 20 with one hand and the lower portion thereof with the other hand in the vicinity of the edge between the front surface portion 15F and one of the side surface portions 15S, and pull at the easy-to-tear portion 20 in the opposite directions as indicated by the arrows in FIG. 3(a). As a result, the sheet material of the packaging bag 10 is torn at the easy-to-tear portion 20, and the packaging bag 10 can be separated into an upper section (lid section) 10a on the upper side and a lower section (main body section) 10b on the lower side (FIG. 3(b)). The upper section 10a may be removed or left placed on the lower section 10b after being separated, and may be removed each time the absorbent article 1 is taken out. In the lower section 10b, the row 1a of the absorbent articles 1, 1, ... is accommodated, and the upper portions of the row 1a of the absorbent articles 1, 1, ... are exposed, so that the absorbent articles 1 can be easily taken out.

The position in the top-bottom direction where the easy-to-tear portion 20 is provided is preferably above the center in the top-bottom direction of the entire packaging bag 10. Further, it is preferable that the easy-to-tear portion 20 be formed below the center of the upper section 10a in the top-bottom direction, that is, the easy-to-tear portion 20 is formed in the second portion from the top when the entire packaging bag 10 is divided into four equal parts in the top-bottom direction. Thus, after the packaging bag 10 is torn open, the absorbent articles 1, 1, ... accommodated in the remaining lower section 10b can be picked up and taken out without touching the packaging bag 10, and the absorbent articles 1, 1, ... can be maintained in an upright state, and the absorbent articles 1, 1, ... can be prevented from being excessively exposed from the packaging bag 10.

In the example illustrated in FIGS. 1 to 3, the easy-to-tear portion 20 is continuously formed over the entire surrounding surface portion 15, but may be formed only on a part of the surface of the surrounding surface portion 15. For example, the easy-to-tear portion 20 may be formed on three surfaces, i.e., the front surface portion 15F and the side surface portions 15S and 15S, and may not be formed on the rear surface portion 15B. Thus, the upper section 10a illustrated in FIG. 3(a) is not completely separated, and can be connected to the lower section 10b at the rear surface portion 15B. In this case, a fold line along a line connecting a terminal position of the easy-to-tear portion 20 at the boundary between one side surface portion 15S and the rear surface portion 15B and a terminal position of the easy-to-tear portion 20 at the boundary between the other side surface portion 15S and the rear surface portion 15B is formed in the rear surface portion 15B, and the upper section 10a is pivotable at the fold line. The upper section 10a falls to the rear side, and the absorbent articles 1, 1, ... can be thereby exposed.

The easy-to-tear portion 20 may not necessarily be continuous, and may include a discontinuous portion in one plane and be separated into a plurality of portions in the lateral direction, for example. In this case, the packaging bag 10 may be used without being torn at the discontinuous portion, or the discontinuous portion may be torn following the tearing of the continuous portion (weakened portion) of the easy-to-tear portion 20. A plurality of the easy-to-tear portions 20 may be formed in proximity to each other in the top-bottom direction. In this case, since a plurality of starting points for separating the upper section and the lower section of the packaging bag 10 are formed in the top-bottom direction, the packaging bag 10 can be more easily torn and opened. In the case of a plurality of easy-to-tear portions close to each other in the top-bottom direction, the length is measured for the plurality of easy-to-tear portions 20 as a whole.

### (Relationship between Easy-to-tear portion and Inside visible region)

Users want to find the easy-to-tear portion 20 immediately when opening the packaging bag 10, but since the packaging bag 10 according to the present embodiment is colored, the easy-to-tear portion 20 is often inconspicuous in the case where it is formed in a colored region. In contrast, as illustrated in FIGS. 1 and 2, in the present embodiment, the easy-to-tear portion 20 overlaps the inside visible region 30. As described above, the inside visible region 30 is a region having high transparency, and therefore, in such a region having high transparency, reflection at the easy-to-tear portion 20, which is a weakened linear portion, is easily recognized. Therefore, users can easily find the position of the easy-to-tear portion 20. Since the inside visible region 30 itself is conspicuous with respect to the colored region 35, users can easily find the inside visible region 30, and can easily find the easy-to-tear portion 20 in a limited region, that is, the inside visible region 30. In this way, the position of the easy-to-tear portion can be easily found, and labor or time required for opening the packaging bag 10 can be reduced.

From the viewpoint of making it easy to find the easy-to-tear portion 20, the portion of the easy-to-tear portion 20 overlapping the inside visible region 30 may be preferably 50% or more, more preferably 60% or more, further preferably 80% or more, and further preferably 90% or more of the entire length of the easy-to-tear portion 20 (or a sum of lengths of the easy-to-tear portions 20 in the case where a plurality of easy-to-tear portions 20 are discontinuously formed). Further, as illustrated in FIGS. 1 and 2, it is preferable that the portion of the easy-to-tear portion 20 overlapping the inside visible region 30 be 100% of the total length of the easy-to-tear portion 20, in other words, the entire easy-to-tear portion 20 be formed in the inside visible region 30. In the front surface portion 15F which is usually first seen by users, the portion of the easy-to-tear portion 20 overlapping the inside visible region 30 may be 50% or more, preferably 50% or more, more preferably 60% or more, further preferably 80% or more, and still further preferably 90% or more of the entire front surface portion 15F of the easy-to-tear portion 20, and the entire easy-to-tear portion 20 is preferably within the inside visible region 30 in the front surface portion 15F.

The appearance color of the absorbent articles to be accommodated (or the color of the packaging sheet for packaging the absorbent articles in the case where the absorbent articles are individually packaged, or the color of the absorbent article itself in the case where the absorbent articles are not individually packaged) is not also limited to a particular color. The appearance color of the absorbent articles may be a color recognized as, for example, brown, black, gray, beige, etc. Furthermore, the chroma in the Munsell color system may be preferably 8 or less, and more preferably 6 or less. The combination of the appearance color of the absorbent articles and the color applied to the packaging bag (the color of the colored region 35) is also not limited to a particular combination. Herein, in the case where the appearance color of the absorbent articles to be accommodated and the color applied to the packaging bag are selected in such a combination that the difference between the colors is immediately recognized by users, for example, in the case where the colors are selected so that the color difference ΔE between the colors is 4 or more, the appearance color of the absorbent articles becomes visible through the inside visible region 30, and thus a user can easily find the inside visible region 30. Therefore, the easy-to-tear portion 20 at least partially overlapping the inside visible region 30 can also be easily found, and the effect of reducing the labor or time required to open the packaging bag can be improved. The color difference ΔE is a color difference ΔE in the CIE1976 L*a*b* color space, and ΔE = [(ΔL*)² + (Δa*)² + (Δb*)²]^{1/2}.

On the other hand, from a viewpoint differing from the above, the appearance color of the absorbent articles accommodated and the color applied to the packaging bag can be selected in combination such that the difference in color between them is not immediately recognized by users, for example, they can be selected such that the color difference ΔE between them is less than 4. In this case, for example, in a state where the packaging bag is torn open and the upper section thereof is removed to expose the upper portions of the absorbent articles (FIG. 3(b)), in a side view for example, the boundary between the packaging bag and the absorbent articles is difficult to see, and the appearance color of the absorbent articles can be made inconspicuous with respect to the color of the packaging bag. Therefore, for example, even in the case where the absorbent article packaged product is placed in the living room, the presence of the absorbent article packaged product is less likely to be noticed.

In addition, in the case where a plurality of inside visible regions 30 are separately formed, the plurality of inside visible regions 30 may include an overlapping inside visible region (also referred to as an "overlapping region") that at least partially overlaps the easy-to-tear portion 20 and a non-overlapping inside visible region (also referred to as a "non-overlapping region") which does not overlap the easy-to-tear portion 20. The non-overlapping region does not have a function of making the easy-to-tear portion conspicuous, but is used to meet the needs of users who want to check whether or not the absorbent articles are actually accommodated, users who want to partially check the appearance (thickness, color, etc.) of the accommodated absorbent articles, and the like. For this reason, the non-overlapping region may not have an elongated shape like the inside visible region 30 illustrated in FIG. 1, etc. The non-overlapping region may have a shape in which the ratio of the maximum length in the top-bottom direction to the maximum length in a direction orthogonal to the top-bottom direction (the left-right direction or the front-rear direction) is about 0.8:1.2 to 1.2:0.8, for example. The non-overlapping region preferably has a size and a shape that fit within a circle having a radius of 600 mm, for example. The shape may be a circle, an ellipse, a polygon such as a rectangle or a triangle, or other shapes such as a heart shape or a drop shape.

As described above, in the case where the appearance color of the absorbent articles accommodated and the color applied to the packaging bag are selected in combination such that the difference in color is immediately recognized by users, the non-overlapping region can be easily confirmed, and thus a desired design can be added to the packaging bag by selecting the shape of the non-overlapping region, which is preferable from the viewpoint of improving design quality. In the case where the non-overlapping region is colored to be partially opaque or translucent to draw a picture, characters, or the like using such an opaque or translucent portion, the outline of such a picture, characters, or the like becomes clear, and desired information or design can be included in the non-overlapping region.

### (Modified Examples)

Hereinafter, modified examples of the absorbent article packaging bag 10 according to the first embodiment will be described. The basic configurations, functions, and usages of the packaging bag 10 according to the modified examples described below are the same as those of the example illustrated in FIGS. 1 and 2. As described below, in the modified examples, the surrounding surface portion 15 is provided with the easy-to-tear portion 20, and the easy-to-tear portion 20 at least partially overlaps the inside visible region 30.

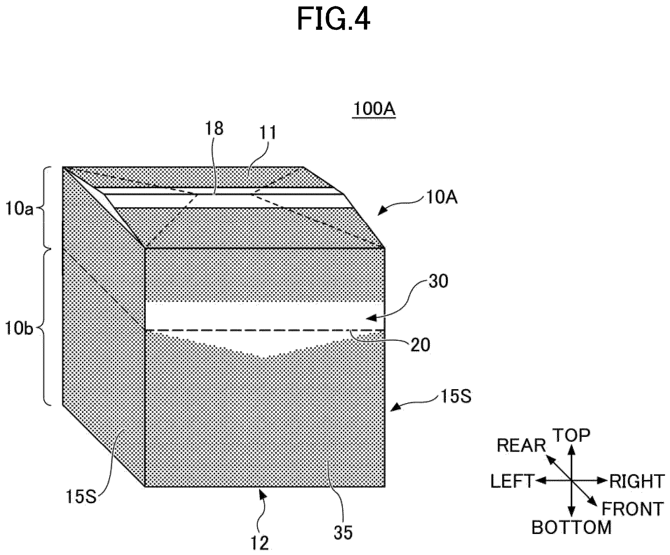

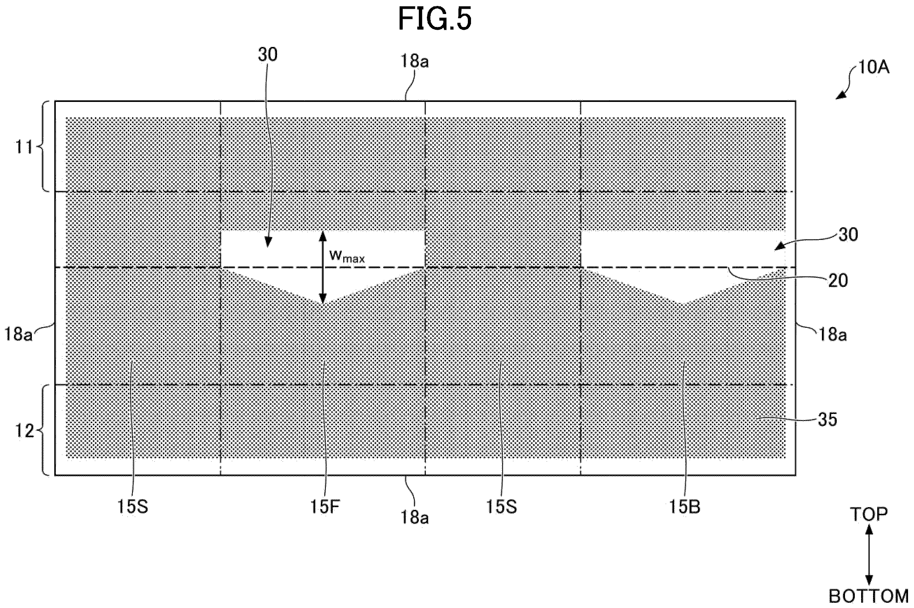

FIG. 4 is a perspective view of an absorbent article packaged product 100A including an absorbent article packaging bag 10A and absorbent articles accommodated in the packaging bag 10A according to a modified example (the absorbent articles are not illustrated). FIG. 5 is a development view of the packaging bag 10A illustrated in FIG. 4. The packaging bag 10A illustrated in FIGS. 4 and 5 is different from the packaging bag 10 (FIGS. 1 and 2) in the size and shape of the inside visible region 30. In the packaging bag 10A, the inside visible region 30 is formed in the front surface portion 15F and the rear surface portion 15B, and is not formed in the side surface portions 15S and 15S. Since the front surface portion 15F is usually the most visible portion of the surface of the packaging bag 10, it is preferable to make the easy-to-tear portion 20 conspicuous in the front surface portion 15F. As in the present embodiment, in the case where each of the front surface portion 15F and the rear surface portion 15B has a larger area than the side surface portion 15S, the inside visible region 30 overlapping with the easy-to-tear portion 20 is provided in the front surface portion 15F and the rear surface portion 15B, each of which has a large area, so that the easy-to-tear portion 20 can be easily found.

In the packaging bag 10A, the inside visible region 30 is not provided in the side surface portions 15S and 15S, and thus, while the ease of finding the easy-to-tear portion 20 is maintained, the colored region 35 is increased, and it is possible to reduce embarrassment caused by the absorbent articles 1 being conspicuous.

In the packaging bag 10A, the inside visible region 30 is not rectangular unlike the inside visible region 30 illustrated in FIGS. 1 and 2, but has an elongated shape with a larger width at the center in the longitudinal direction of the elongated inside visible region 30. In the case where the width of the inside visible region 30 varies at places as described above, the maximum width wₘₐₓ of the inside visible region 30 may be 10 to 30 mm. In this way, the shape of the inside visible region 30 having a partially different width is easily noticeable, and thus users can easily find the inside visible region 30. When the inside visible region 30 is focused, the easy-to-tear portion 20 overlapping the inside visible region 30 can be easily found, and therefore, the easy-to-tear portion 20 can be immediately found at the time of opening the bag, and labor or time for opening can be saved. In addition, the design of the packaging bag 10 is improved.

In the packaging bag 10A, the inside visible region 30 has a pentagon shape in which an upper portion of a rectangle and an isosceles triangle having a base equal to a long side of the rectangle are joined together, but the shape of the inside visible region 30 is not limited to a particular shape. For example, the shape may be a polygon other than a quadrangle and a pentagon, an ellipse, or another shape that is elongated along the direction in which the easy-to-tear portion 20 extends. In addition, in one inside visible region 30, there may be two or more places where the width is larger than the other places. The outline of the inside visible region 30 is not limited to a straight line, and may be a curved line, for example, a wave shape.

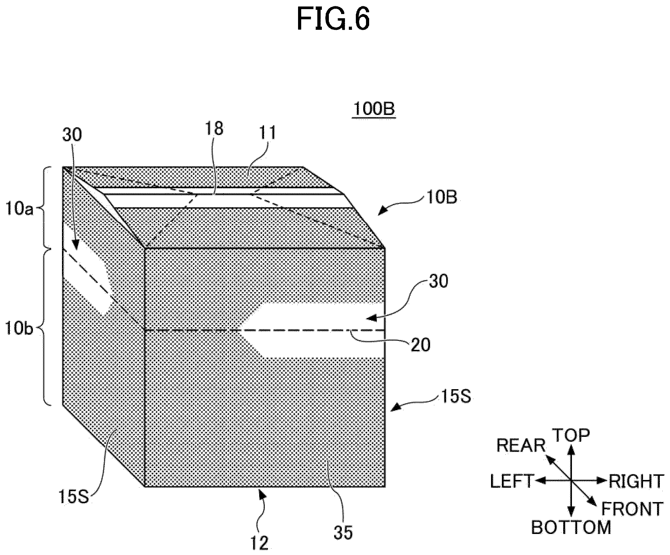

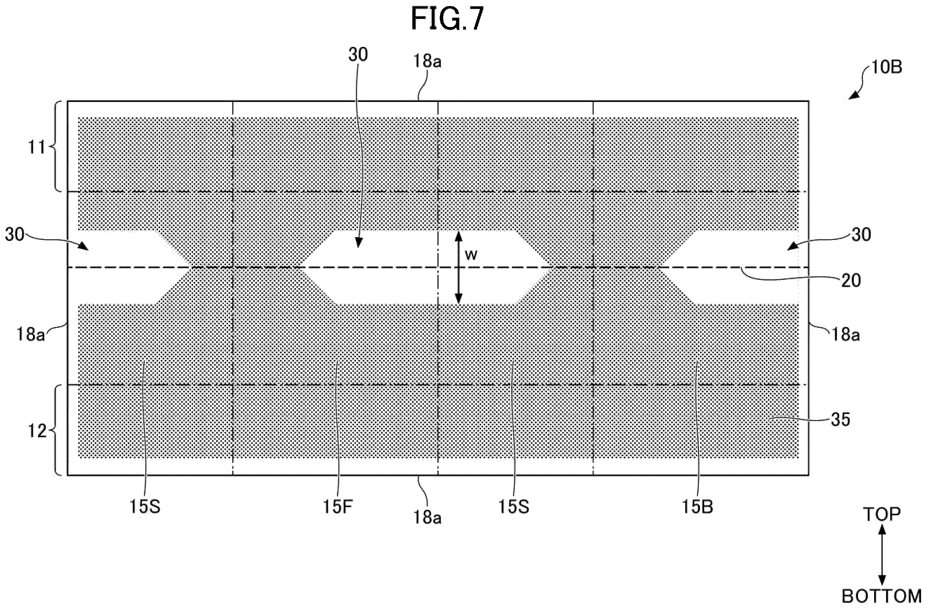

FIG. 6 is a perspective view of an absorbent article packaged product 100B including a packaging bag 10B according to another modified example and absorbent articles accommodated in the packaging bag 10B (the absorbent articles are not illustrated). FIG. 7 is a development view of the packaging bag 10B illustrated in FIG. 6. The packaging bag 10B illustrated in FIGS. 6 and 7 is different from the packaging bag 10 (FIGS. 1 and 2) in the size and shape of the inside visible region 30. The packaging bag 10B is not configured in such a manner that one inside visible region 30 is continuous so as to surround the packaging bag 10 as illustrated in FIGS. 1 and 2. In the packaging bag 10B, the inside visible region 30 is divided into two, and each inside visible region 30 is formed across two adjacent surrounding surface portions of the surrounding surface portion 15. To be more specific, one of the inside visible regions 30 is formed across the front surface portion 15F and one side surface portion 15S, and the other inside visible region 30 is formed across the rear surface portion 15B and the other side surface portion 15S. Since the boundary portion between the two adjacent surrounding surface portions is easily noticed, when the inside visible region 30 is formed across the two adjacent surrounding surface portions, the inside visible region 30 can be easily found, and thus the easy-to-tear portion 20 overlapping the inside visible region 30 can be more easily found. When the bag is opened, the bag is pulled by holding the vicinity of the boundary portion between the two adjacent surrounding surface portions 15 and 15 (FIG. 3(b)), the easy-to-tear portion 20 is easily torn, and the bag is easily opened. Therefore, the inside visible region 30 formed across two adjacent surrounding surface portions 15 is preferable also from the viewpoint of guiding users to open the bag in the vicinity of the boundary between the two adjacent surrounding surface portions.

In the packaging bag 10B, a portion of the easy-to-tear portion 20 overlapping the inside visible region 30 is 50% or more of the entire easy-to-tear portion 20.

The inside visible region 30 of the packaging bag 10B also has an elongated shape, but the end portion in the longitudinal direction has a protruding shape in the direction in which the easy-to-tear portion 20 extends. More specifically, the inside visible region 30 has a shape formed by combining a triangle having a vertex on the easy-to-tear portion 20 and a rectangle to which the triangle is connected at the ends in the longitudinal direction. The inside visible region 30 of the packaging bag 10B is shaped to be narrower toward the outside in the longitudinal direction. Such a shape can remind users of an arrow, and therefore, can make users be more strongly aware of the direction in which the easy-to-tear portion 20 extends (the direction in which users open the bag).

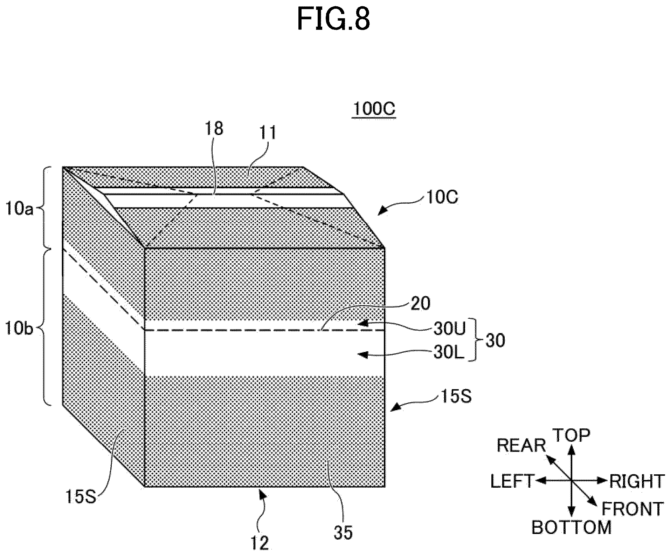

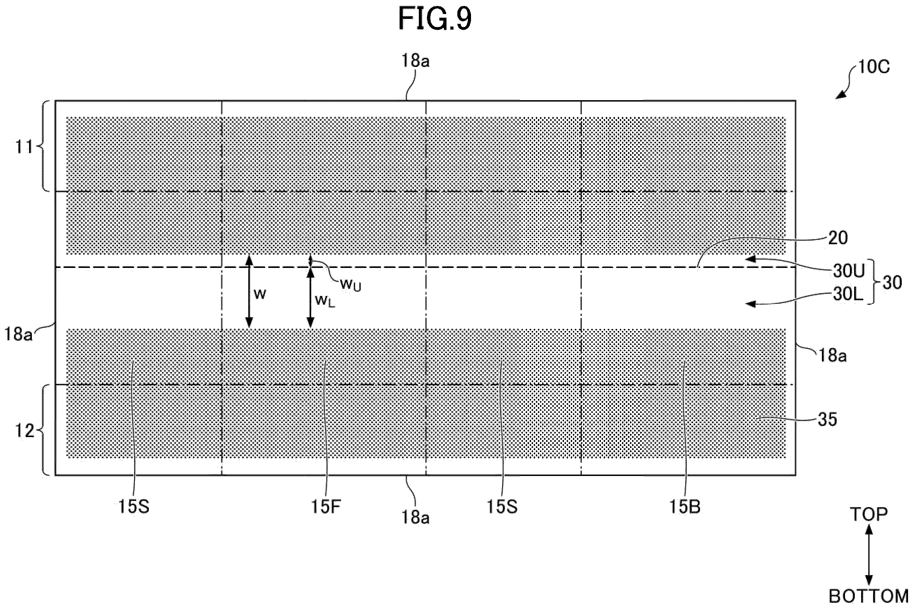

FIG. 8 is a perspective view of an absorbent article packaged product 100C including a packaging bag 10C according to another modified example and absorbent articles accommodated in the packaging bag 10C (the absorbent articles are not illustrated). FIG. 9 is a development view of the packaging bag 10C illustrated in FIG. 8. The packaging bag 10C illustrated in FIGS. 8 and 9 is different from the packaging bag 10 (FIGS. 1 and 2) in the size and arrangement of the inside visible region 30.

In the packaging bag 10 (FIGS. 1 and 2), the easy-to-tear portion 20 extends in the center of the inside visible region 30 in the top-bottom direction. In contrast, in the packaging bag 10C illustrated in FIGS. 8 and 9, the easy-to-tear portion 20 extends in a position close to the upper side of the inside visible region 30. Alternatively, in the case where the inside visible region 30 is divided at the easy-to-tear portion 20, suppose if the upper portion is referred to as an "upper inside visible region 30U" and the lower portion is referred to as a "lower inside visible region 30L", the width (the length in the top-bottom direction) w_{L} of the lower inside visible region 30L is larger than the width (the length in the top-bottom direction) w_{U} of the upper inside visible region 30U. In the front surface portion 15F, the area of the lower inside visible region 30L is larger than that of the upper inside visible region 30U. Furthermore, the area of the lower inside visible region 30L is larger than that of the upper inside visible region 30U in the entire surrounding surface portion 15 (15F, 15B, 15S, 15S).

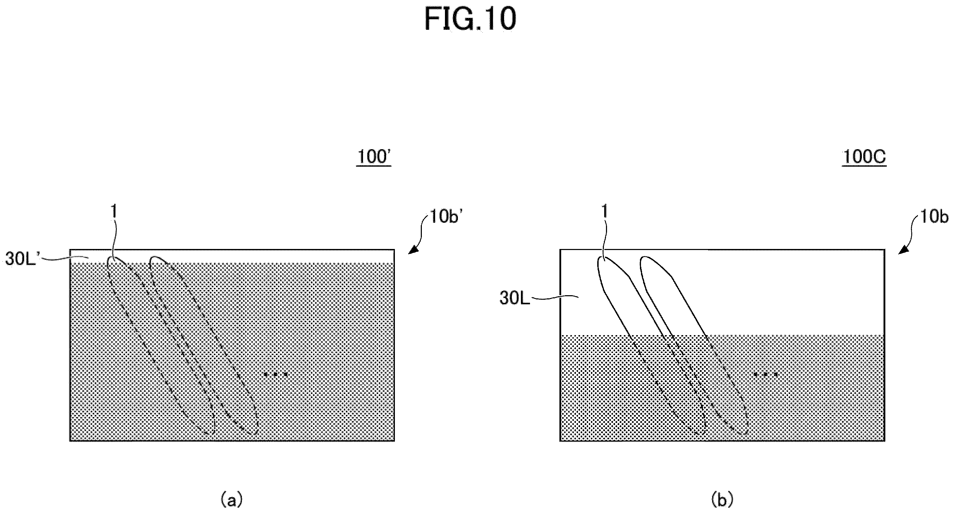

Advantages of the packaging bag 10C will be described with reference to FIG. 10. FIG. 10 is a front view illustrating a state where the absorbent articles 1, 1, ... are accommodated in the lower section 10b after the packaging bag is torn open at the easy-to-tear portion 20 and the upper section 10a is removed. FIG. 10(a) illustrates an example where an absorbent article packaged product 100' has a lower inside visible region 30L' having a small area or has a lower section 10b' in which a width of a lower inside visible region 30' is small. On the other hand, FIG. 10(b) illustrates the lower section 10b of the absorbent article packaged product 100C (FIGS. 8 and 9), which remains after the packaging bag 10C is torn open at the easy-to-tear portion 20. In the example illustrated in FIG. 10(b) (the lower section 10b of the packaging bag 10C), the area of the lower inside visible region 30L is larger than that in FIG. 10(a), and thus the inside of the bag is easily visible in a front view, for example. For this reason, the presence of the absorbent articles 1, 1, ... or the number of the absorbent articles 1, 1, ... can be easily checked even when the absorbent articles 1, 1, ... in an upright state become slanted.

As described above, according to the packaging bag 10C, it is possible to easily recognize the state of the absorbent articles 1, 1, ... accommodated in the lower section 10b that remains after opening the bag when viewed from the surrounding surface (at least one of a front view, a side view, or a rear view), while the effect of the present embodiment that the easy-to-tear portion 20 is easily found is maintained through securing a sufficient width w of the entire inside visible region 30.

The width w_{L} of the lower inside visible region 30L may be set to 15 to 25 mm regardless of the relationship in size between the width w_{L} of the lower inside visible region 30U and the width w_{U} of the upper inside visible region 30L. This makes it easy to check the state of the absorbent articles 1, 1, ... through the surrounding surface portion 15, and also makes it possible to avoid the lower inside visible region 30L in the lower section 10b that remains after opening of the bag from becoming excessively large and the entire absorbent articles 1 from being viewed.

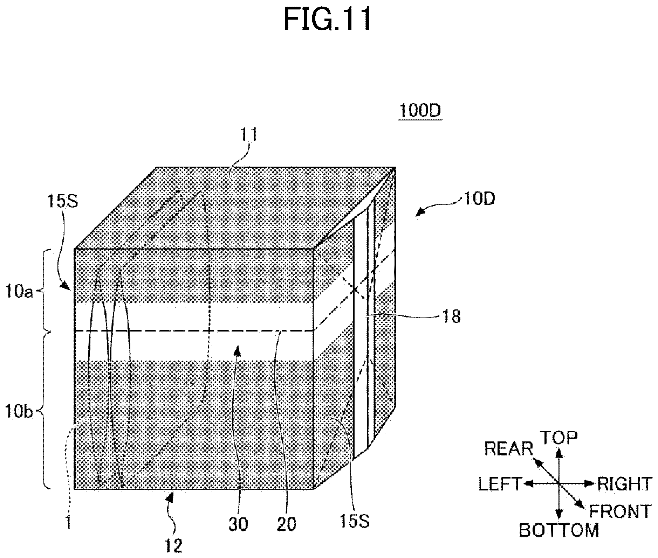

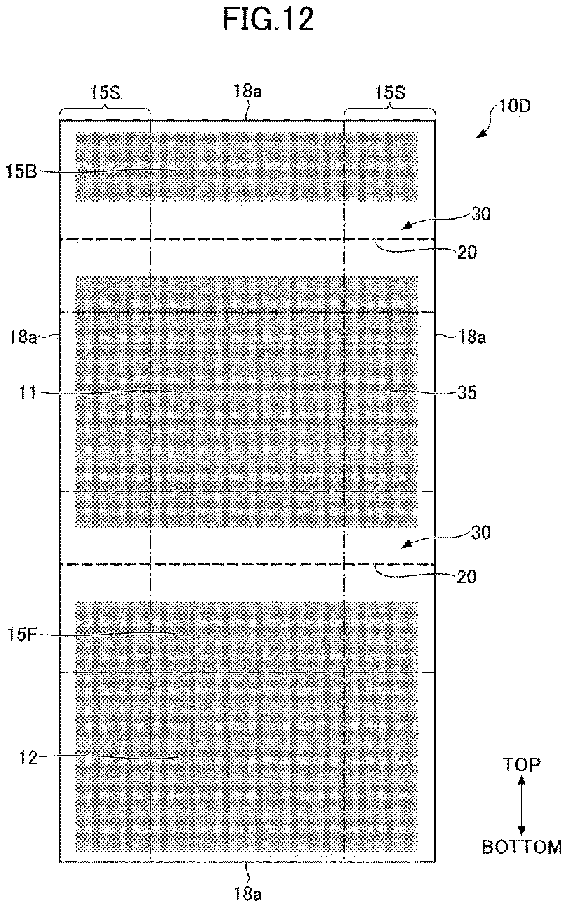

FIG. 11 is a perspective view of an absorbent article packaged product 100D including a packaging bag 10D according to another modified example and absorbent articles packaged in the packaging bag 10D (the absorbent articles are not illustrated). FIG. 12 is a development view of the packaging bag 10D illustrated in FIG. 11. The packaging bag 10D illustrated in FIGS. 11 and 12 accommodates the absorbent articles 1, 1, ... in an upright state, but differs from the above-described packaging bag 10 (FIGS. 1 and 2), packaging bag 10A (FIGS. 4 and 5), packaging bag 10B (FIGS. 6 and 7), and packaging bag 10C (FIGS. 8 and 9) in the position where the packaging bag 10D is sealed, namely the position of the joined portion 18. In the packaging bag 10D, the joined portion 18 is formed in the side surface portion 15S.

As described above, in this embodiment, the sealing position of the packaging bag is not limited to a particular position, but the easy-to-tear portion 20 is provided in the surrounding surface portion 15 arranged to surround the virtual line in the top-bottom direction, with the direction in which the absorbent articles 1 stand as the top-bottom direction, and the easy-to-tear portion 20 at least partially overlaps the inside visible region 30, regardless of forms of packaging.

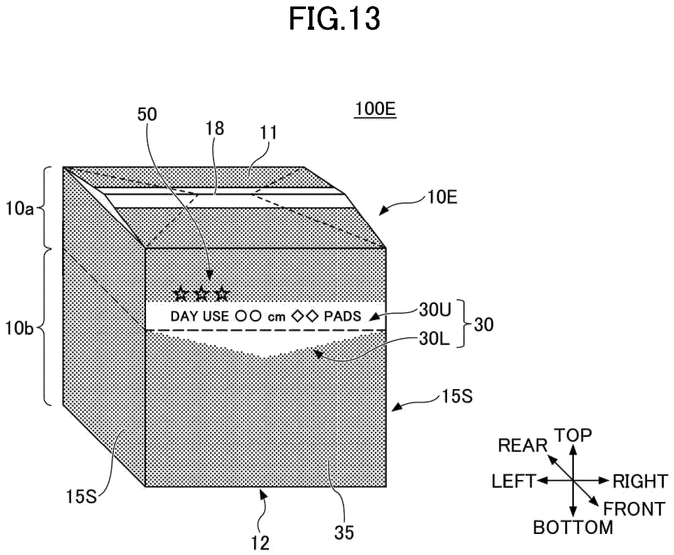

FIG. 13 is a perspective view of an absorbent article packaged product 100E including a packaging bag 10E according to another modified example and absorbent articles accommodated in the packaging bag 10E (the absorbent articles are not illustrated). The packaging bag 10E illustrated in FIG. 13 is the packaging bag 10A (FIGS. 4 and 5) on which article-related information 50 (information regarding the absorbent articles) is indicated. Herein, the article-related information 50 may be one or more of a product name, a manufacturer name, distributor name, product specifications or product characteristics of the absorbent articles 1, such as a product length, with or without wings, thickness (regular type, slim type, etc.), absorption amount, recommended use (daytime, overnight, maxi), fit (fluffy type, good breathability), number of pads accommodated in a packaging bag, and usage. The article-related information 50 may be characters, drawings, photographs, codes, symbols, logo marks, or the like.

As illustrated in FIG. 13, the article-related information 50 is preferably added to the front surface portion 15F so that users can easily distinguish a product from other products at the time of purchase. Furthermore, in order to prevent confusion of users, it is preferable that the article-related information 50 be added to the front surface portion 15F and be not added to the rear surface portion 15B, and for example, it is more preferable that the main information be added only to the front surface portion 15F. In the present embodiment, the article-related information 50 is added above the easy-to-tear portion 20. In the case where the packaging bag 10E is opened by tearing the easy-to-tear portion 20, a portion above the easy-to-tear portion 20 corresponds to the upper section 10a (FIG. 3(b)). Thus, it is possible to eliminate or minimize the article-related information left in the lower portion 10b after the upper section 10a has been separated off and removed or pivoted to the rear surface portion 15B. Therefore, even in the case where the packaging bag 10E is opened and the portion below the easy-to-tear portion 20, namely the upper section 10a (FIG. 3(b)) in which the absorbent articles 1, 1, ... are accommodated is placed in a conspicuous place, it is difficult to immediately recognize that the accommodated articles are absorbent articles. Such an effect (being less recognizable) can be improved by the configuration (color, material) of the packaging sheet for packaging the absorbent article 1, coloring applied to the packaging bag, and the like. From the viewpoint of further improving less-recognizable properties, it is more preferable that article-related information be not added to a portion below the easy-to-tear portion 20 (lower section 10b).

### <Second Embodiment>

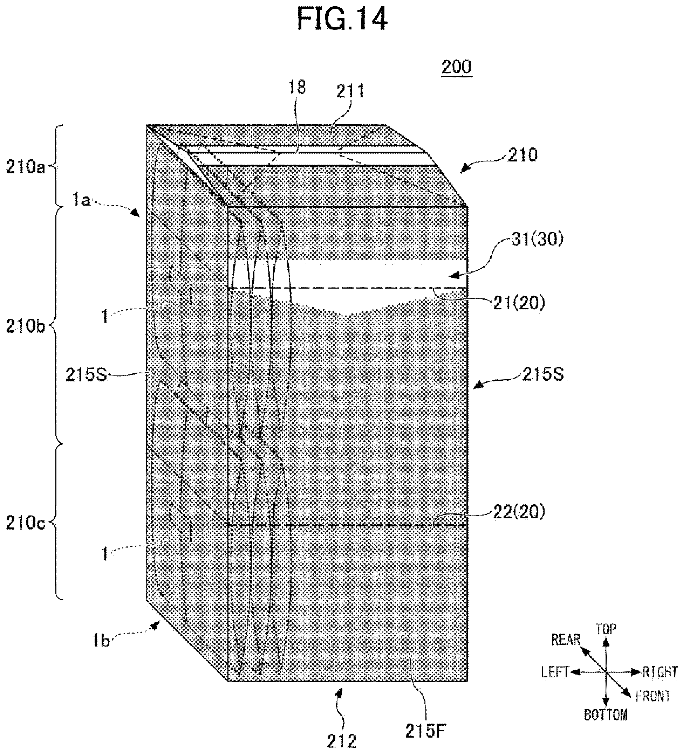

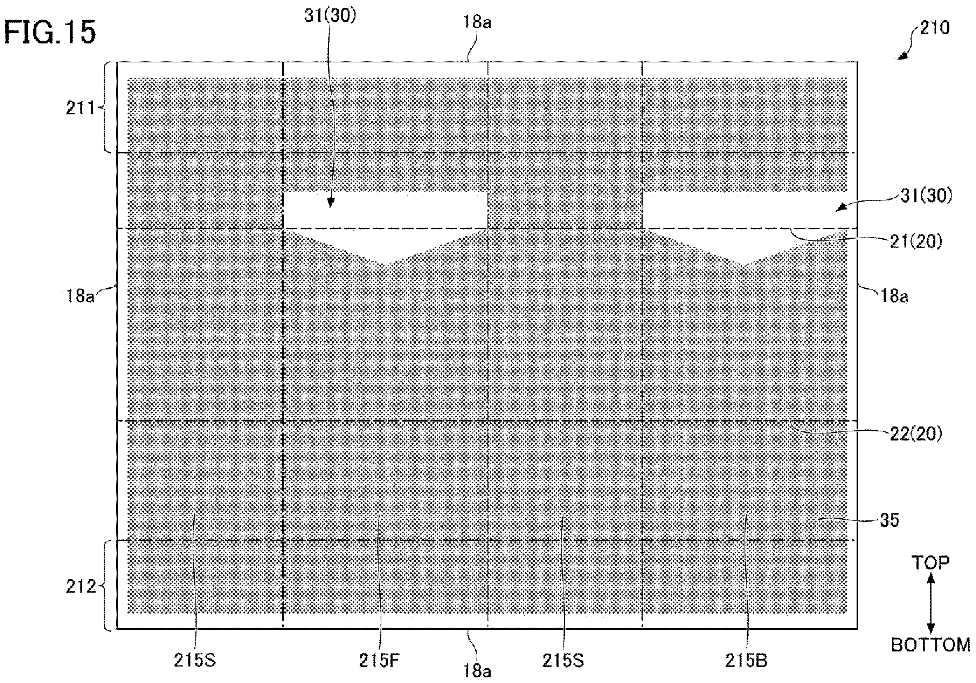

FIG. 14 illustrates an absorbent article packaged product 200 including an absorbent article packaging bag 210 according to the second embodiment and a plurality of absorbent articles 1 accommodated in the absorbent article packaging bag 210. FIG. 15 is an unfolded-open view of the absorbent article packaging bag 210 illustrated in FIG. 14. The basic configurations and functions of the elements included in the second embodiment may be the same as the configurations and functions of the elements included in the first embodiment unless otherwise specified.

The packaging bag 210 according to the present embodiment is also colored similarly to the packaging bag 10 according to the first embodiment, and has a substantially rectangular parallelepiped shape having a top surface portion 211, a bottom surface portion 212, and a surrounding surface portion 215 (a front surface portion 215F, a rear surface portion 215B, and side surface portions 215S and 215S) connecting the top surface portion 211 and the bottom surface portion 212. In the packaging bag 210 according to the present embodiment, similarly to the first embodiment, a plurality of absorbent articles 1, 1, ... are accommodated in an upright state and aligned in a stacked manner in the thickness direction of the absorbent article 1, but the absorbent articles 1, 1, ... accommodated in the packaging bag 210 are in two or more stages in the top-bottom direction. In other words, a plurality of rows of the plurality of absorbent articles 1, 1, ..., which are aligned to overlap in the thickness direction, are arranged in the top-bottom direction. In the example illustrated in FIG. 14, two rows of a first absorbent article row 1a which is a row of the absorbent articles 1, 1, ... on the upper side and a second absorbent article row 1b which is a row of the absorbent articles 1, 1, ... on the lower side are accommodated.

In the case where two rows of absorbent articles are accommodated in the top-bottom direction, the size of the packaging bag 210 according to the second embodiment may be about 80 to 300 mm in the left-right direction, 60 to 200 mm in the front-rear direction, and 100 to 400 mm in the top-bottom direction.

Similarly to the first embodiment, the packaging bag 210 of the present embodiment is also provided with the easy-to-tear portion 20 formed in the surrounding surface portion 215 (one or more surface portions of the front surface portion 215F, the rear surface portion 215B, and the side surface portions 215S and 215S). The easy-to-tear portion 20 in the present embodiment includes a first easy-to-tear portion 21 formed in a range in the top-bottom direction in which the first absorbent article row 1a is accommodated, and a second easy-to-tear portion 22 formed in a range in the top-bottom direction in which the second absorbent article row 1b is accommodated. Therefore, the first easy-to-tear portion 21 and the second easy-to-tear portion 22 are separated from each other in the top-bottom direction.

To start using, the packaging bag 210 is torn into an upper section and a lower section at the uppermost easy-to-tear portion, namely at the first easy-to-tear portion 21 in the example illustrated in the drawing, and the absorbent article packaged product 200 is opened. The tearing at the first easy-to-tear portion 21 can be performed by, for example, taking the vicinity of the edge between the adjacent surrounding surface portions 15S and 15S and pulling the vicinity of the edge in the opposite directions, similarly to the tearing at the easy-to-tear portion 20 according to the first embodiment described with reference to FIG. 3.

For example, in the case where the first easy-to-tear portion 21 is formed in an annular shape over the surrounding surface portion 15, the upper portion (lid section) 210a above the first easy-to-tear portion 21 can be removed by tearing open the packaging bag 210, and the upper portion of the first absorbent article row 1a is exposed. After finishing the absorbent articles 1, 1, ... included in the first absorbent article row 1a, users can tear the packaging bag 210 into an upper section and a lower section at the second easy-to-tear portion 22. The middle section 210b and the lower section 210c of the packaging bag 210 are separated by tearing the second easy-to-tear portion 22, and the middle section 210b can be removed thereafter. Thus, the upper portion of the second absorbent article row 1b is exposed, and the absorbent articles 1 in the second absorbent article row 1b can be easily taken out. Users can also take out the absorbent articles 1 of the second absorbent article row 1a by putting a hand into the packaging bag 210 without tearing the second easy-to-tear portion 22 after finishing the absorbent articles 1, 1, ... of the first absorbent article row 1b. However, by removing the middle section 210b through tearing the second easy-to-tear portion 22, the absorbent articles 1 can be easily reached, and the noise generated by the absorbent article 1 and the packaging bag or the sheet materials constituting the packaging bag rubbing against each other when the absorbent article 1 is taken out can be reduced. Furthermore, the packaging bag 210 after the first absorbent article row 1a is finished becomes compact and looks neat.

In the packaging bag 210 according to the present embodiment, similarly to the first embodiment, the inside visible region 30 is formed at least in the surrounding surface portion 15. In the example illustrated in FIGS. 14 and 15, the inside visible region 30 is formed so as to at least partially overlap the first easy-to-tear portion 21. This allows users to easily find the first easy-to-tear portion 21, and it is thus possible to reduce time and effort required for opening the packaging bag 210. When a plurality of rows of absorbent articles are accommodated and the easy-to-tear portions are formed so as to correspond to the respective rows as in the present embodiment, users do not necessarily tear open the packaging bag at all of the plurality of easy-to-tear portions, but usually tear open the packaging bag 210 at the first easy-to-tear portion 21 located at the uppermost position to open the bag. Therefore, it is preferable that the first easy-to-tear portion 21 located at the uppermost position partially overlap the inside visible region 30.

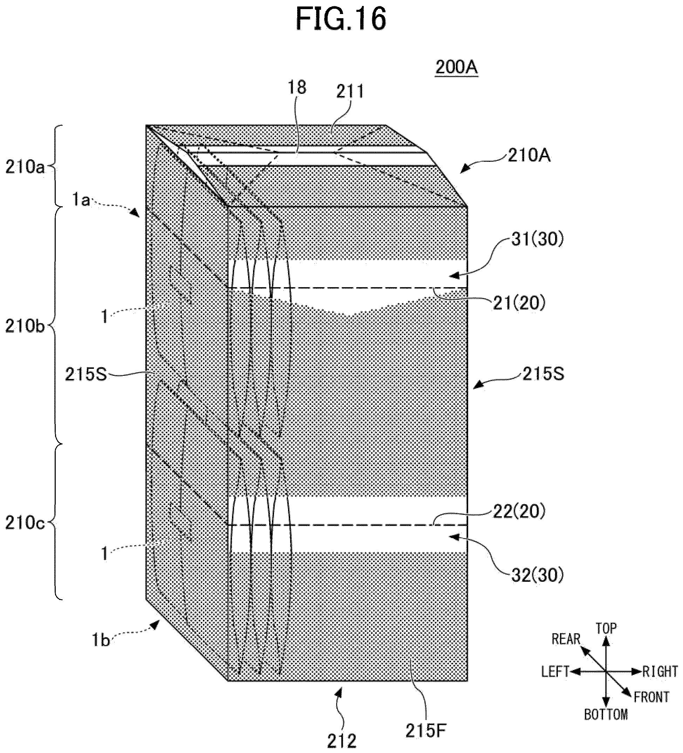

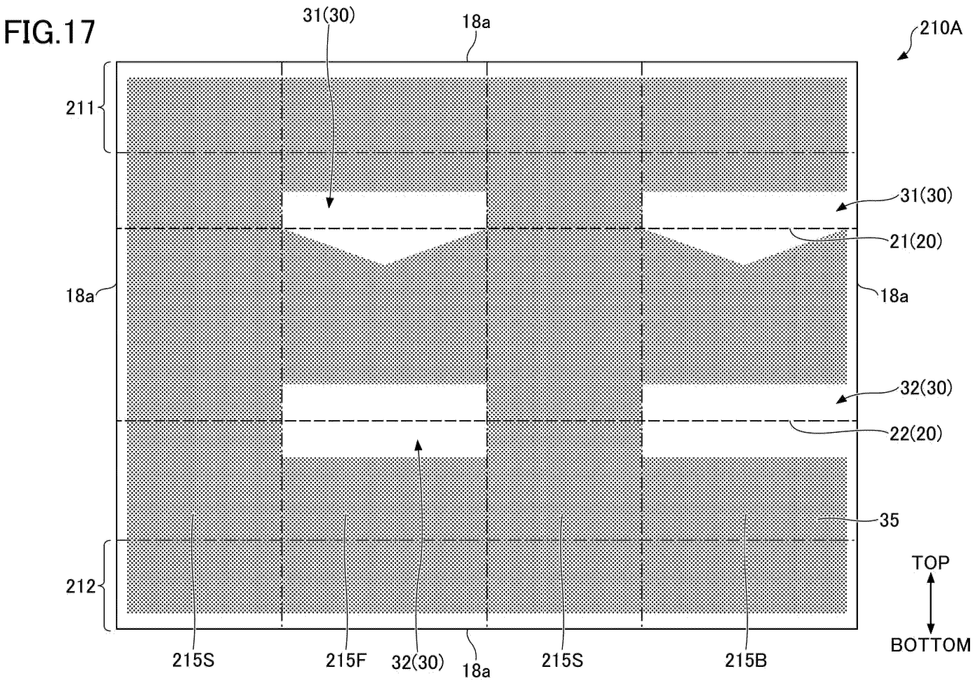

FIG. 16 illustrates an absorbent article packaged product 200A including an absorbent article packaging bag 210A according to a modified example and a plurality of absorbent articles 1 accommodated in the absorbent article packaging bag 210A. FIG. 17 is an unfolded-open view of the absorbent article packaging bag 210A illustrated in FIG. 16. The packaging bag 210A according to the present modified example is different from the packaging bag 210 illustrated in FIGS. 14 and 15 in that a plurality of inside visible regions 30 are formed separately in the upper and lower sections. In other words, in the present example, the inside visible region 30 includes a first inside visible region 31 that at least partially overlaps the first easy-to-tear portion 21 and a second inside visible region 32 that at least partially overlaps the second easy-to-tear portion 22.

In the packaging bag 210A, unlike the packaging bag 210 (FIGS. 14 and 15), the second easy-to-tear portion 22 also overlaps the inside visible region 32 (30), and thus a user can also easily find the second easy-to-tear portion 22. Therefore, it is possible to reduce effort or time not only for tearing the first easy-to-tear portion 21 but also for tearing the second easy-to-tear portion 22 to separate the middle section 210b and the lower section 210c after the first absorbent article row 1a is finished.

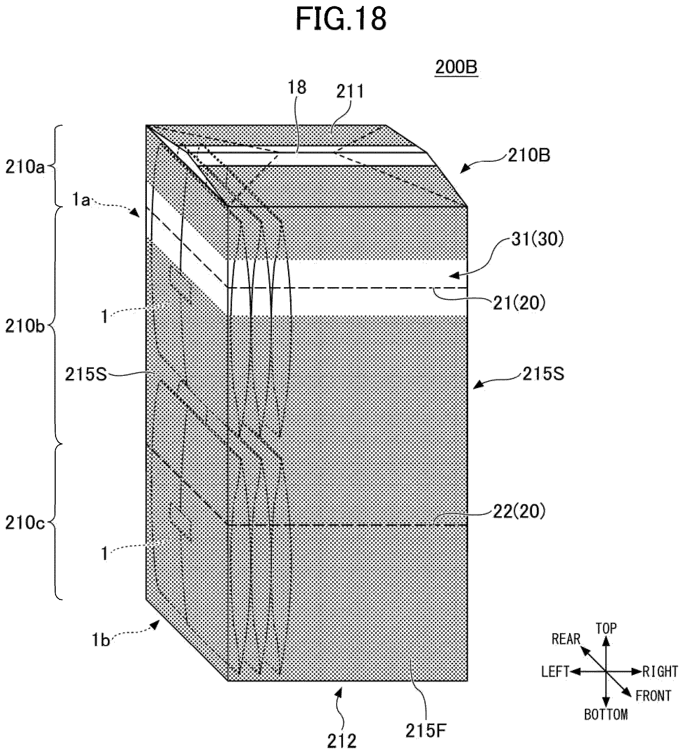

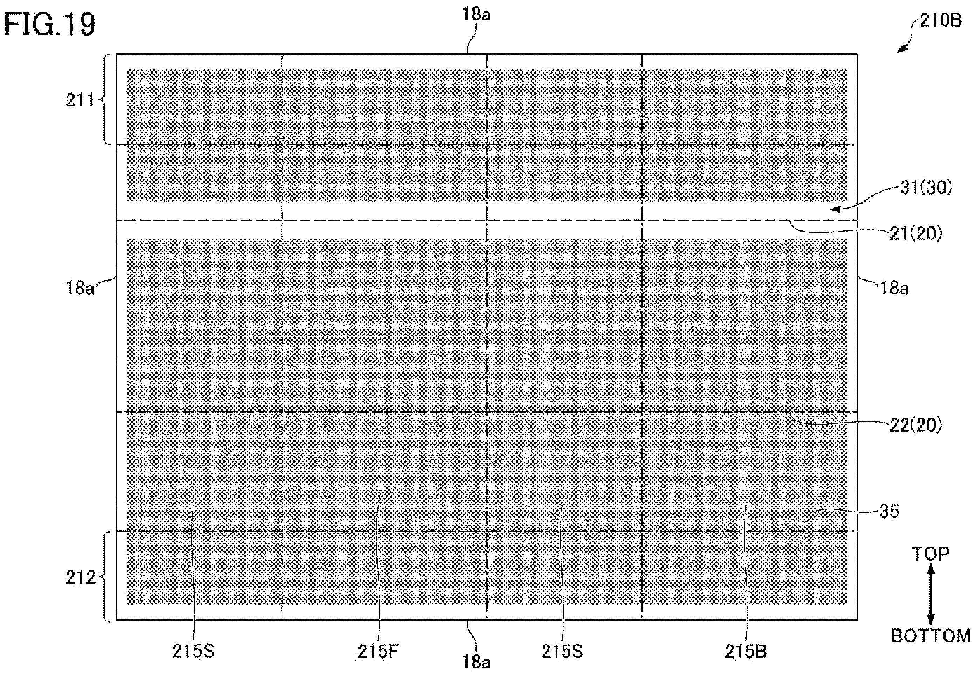

FIG. 18 illustrates an absorbent article packaged product 200B including an absorbent article packaging bag 210B according to a modified example and a plurality of absorbent articles 1 accommodated in the absorbent article packaging bag 210B. FIG. 19 is an unfolded-open view of the absorbent article packaging bag 210B illustrated in FIG. 18. The packaging bag 210B according to the present modified example is different from the packaging bag 210 (FIGS. 14 and 15) in that the inside visible region 30 is formed in an annular shape over the surrounding surface portion 15. In the present modified example, the entire first easy-to-tear portion 21 is formed so as to be included in the inside visible region 30 (the first inside visible region 31). On the other hand, the second easy-to-tear portion 22 does not overlap the inside visible region.

In the packaging bag 210B, the inside visible region 31 (30) overlaps the first easy-to-tear portion 21 located at the uppermost position, similarly to the packaging bag 210 (FIGS. 14 and 15). This makes it possible to make the first easy-to-tear portion 21, which is a part that users usually desire to tear first to open the packaging bag, conspicuous. In the case of the packaging bag 210B, since the inside visible region 31 (30) is formed annularly over the four surrounding surface portions 15, the inside visible region 31 (30) is exposed regardless of the direction in which the absorbent article packaged product 200B is placed, and it is therefore easy to find the inside visible region 31 (30) and the position of the first easy-to-tear portion 21.

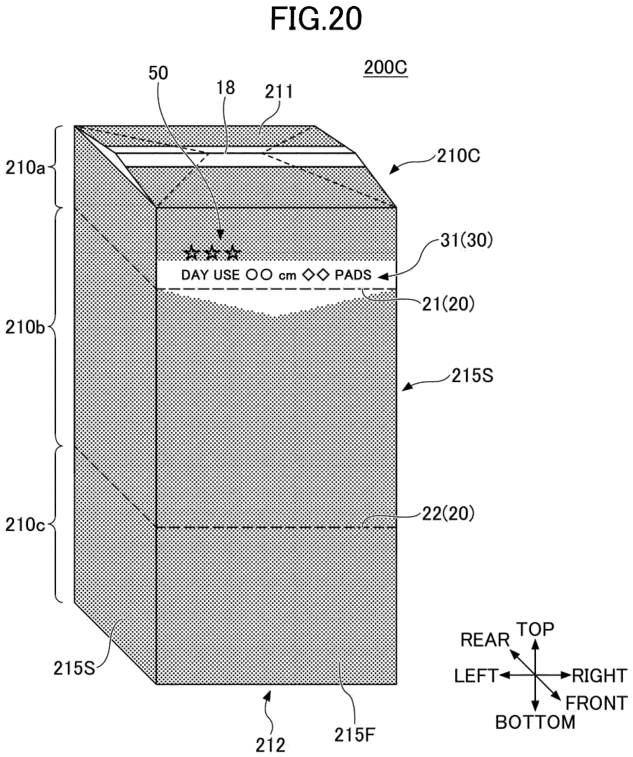

FIG. 20 illustrates an absorbent article packaged product 200B including an absorbent article packaging bag 210C according to another modified example and a plurality of absorbent articles 1 accommodated in the absorbent article packaging bag 210C. The packaging bag 210C is the packaging bag 210 (FIGS. 14 and 15) on which article-related information 50 (information regarding the absorbent articles) is indicated. The article-related information is the same as the information (FIG. 13) described in the first embodiment.

As illustrated in FIG. 20, the article-related information 50 is preferably added to the upper portion 210a above the first easy-to-tear portion 21. When opening the packaging bag, the packaging bag 210C is usually torn open at the first easy-to-tear portion 21 as described above, and the upper section 210a is cut off and removed or is turned down toward the rear surface portion 215B side so that the upper portions of the accommodated absorbent articles are exposed. This can eliminate or minimize the article-related information left in the middle section 210b and the lower section 210c. Therefore, even in the case where the portion below the first easy-to-tear portion 21 (the middle section 210b and the lower section 210c) is placed in a conspicuous place after the packaging bag 210C is opened, it is difficult to immediately recognize that the accommodated article is an absorbent article.

From the viewpoint of making it more difficult to recognize that the accommodated article is an absorbent article, it is preferable that article-related information be not added to at least the lower section 210c, which is likely to be used for a longest period of time over a period of use of the absorbent article packaged product 210C. It is more preferable that article-related information be not added below the first easy-to-tear portion 21 **(i.e.,** the middle section 210b and the lower section 210c) of the packaging bag 210C.

The present invention has been described above based on the embodiments, but the present invention is not limited to these embodiments. The above-described embodiments can be variously changed, modified, replaced, added, deleted, combined, and the like within the scope described in the claims, and these also belong to the technical scope of the present invention.

Specific aspects of the present invention will be described below.

### (Clause 1)

An aspect according to Clause 1 is a colored absorbent article packaging bag that can accommodate a plurality of absorbent articles in an upright state, the packaging bag having a substantially rectangular parallelepiped shape having a top surface portion, a bottom surface portion, and a surrounding surface portion between the top surface portion and the bottom surface portion, the surrounding surface portion having an inside visible region through which part of the absorbent articles is visible, the surrounding surface portion having an easy-to-tear portion with which the surrounding surface portion can be torn in a top-bottom direction, and the easy-to-tear portion at least partially overlapping the inside visible region.

Since the absorbent article packaging bag according to the aspect of the above Clause 1 is colored, it is difficult to view the appearance of the absorbent article or an individual package of the absorbent article inside the bag when viewed from the outside of the bag. Although it is usually felt embarrassing and avoided that the absorbent article or the individual package of the absorbent article be seen by others, a colored packaging bag of the present aspect can reduce such embarrassment. However, there are users who want to check whether or not the absorbent article is actually accommodated, or want to partially check the appearance of the contained absorbent article, and further, users who want to check the absorbent article remaining in the packaging bag after the packaging bag is opened, from the outside of the packaging bag. Such users may find it inconvenient or uncomfortable if the entire packaging bag is completely colored and the contents are not visible at all. In contrast, according to this aspect, since the inside visible region in which part of the absorbent articles can be viewed is formed in at least the surrounding surface portion of the packaging bag, it is possible to check whether the absorbent article is actually accommodated, the partial appearance of the absorbent article, the absorbent article remaining after the packaging bag is opened, and the like, while avoiding the absorbent article in the packaging bag from being immediately recognized at a glance, whenever a need of checking arises.

In this aspect, the surrounding surface portion is provided with the easy-to-tear portion that is tearable in the top-bottom direction. To take out the absorbent article in the packaging bag, a user can tear open the packaging bag at the easy-to-tear portion to form a take-out opening in the packaging bag. However, the easy-to-tear portion formed in the colored packaging bag is often inconspicuous when viewed from the outside of the bag and is difficult to find. Therefore, it takes time and effort to open the packaging bag, which may be stressful for a user. In contrast, in the present aspect, the easy-to-tear portion at least partially overlaps the above-described inside visible region. Since the inside visible region has a high light transmittance, reflection of light at the easy-to-tear portion present in such a region is easily recognized. Therefore, a user can easily find the position of the easy-to-tear portion, and can reduce labor or time required for opening the packaging bag.

### (Clause 2)

In the aspect according to Clause 2, the easy-to-tear portion is formed in a linear shape, and a ratio of a portion of the easy-to-tear portion overlapping the inside visible region to the entire length of the easy-to-tear portion is 50% or more.

According to the aspect of the above Clause 2, the overlapping portion between the easy-to-tear portion and the inside visible region is secured to some extent, and the above effect that users can easily find the position of the easy-to-tear portion can be further improved.

### (Clause 3)

In the aspect according to Clause 3, the easy-to-tear portion is formed in an annular shape in a direction surrounding the absorbent article packaging bag, and the entire easy-to-tear portion is formed within the inside visible region.

According to the aspect of the above Clause 3, since the easy-to-tear portion is annularly continuous, the packaging bag can be easily separated into two sections by tearing the bag, and the absorbent article or the individual package of the absorbent article can be more easily taken out. Further, since the entirety of the easy-to-tear portion is formed within the inside visible region, the above-described effect that users can easily find the position of the easy-to-tear portion can be further improved.

### (Clause 4)

In the aspect according to Clause **4,** the inside visible region is elongated in an orthogonal direction orthogonal to the top-bottom direction and has a shape that is wider at a center in the orthogonal direction.

According to the aspect of the above Clause 4, the width of the inside visible region is partially increased at the center in the longitudinal direction of the inside visible region, and this makes the inside visible region conspicuous. Therefore, the easy-to-tear portion overlapping the inside visible region can be more easily found. In addition, the design of the packaging bag is improved.

### (Clause 5)

In the aspect according to Clause 5, the inside visible region includes an upper inside visible region above the easy-to-tear portion and a lower inside visible region below the easy-to-tear portion, and an area of the lower inside visible region is larger than an area of the upper inside visible region.

According to the aspect of the above Clause 5, when the packaging bag is separated into an upper section and a lower section at the easy-to-tear portion, it is possible to increase the area in which the absorbent article or the individual package of the absorbent article accommodated in the lower section can be viewed in, for example, a front view. Therefore, even when the accommodated absorbent article or the accommodated individual package of the absorbent article in an upright state becomes slanted and is not exposed from the upper edge of the lower portion, the presence of the absorbent article or the individual package of the absorbent article can be checked in a front view.

### (Clause 6)

In the aspect according to Clause 6, information of the absorbent articles is added above the easy-to-tear portion.

According to the aspect of the above Clause 6, when the packaging bag is torn into an upper section (lid section) and a lower section (main body section) at the easy-to-tear portion, and in the case where the upper section is removed, it is possible not to leave the information of the absorbent articles in the lower section or minimize the information of the absorbent articles remaining in the lower section. Therefore, this makes it difficult to immediately recognize that the accommodated article is an absorbent article, even in the case where the absorbent article or the individual package of the absorbent article accommodated only in the lower section is placed at a conspicuous place.

### (Clause 7)

In the aspect according to Clause **7,** two or more absorbent article rows in which a plurality of absorbent articles are arranged in an upright state can be accommodated in the top-bottom direction, the easy-to-tear portion includes a first easy-to-tear portion formed within a range in the top-bottom direction of an uppermost first absorbent article row of the two or more absorbent article rows and a second easy-to-tear portion formed within a range in the top-bottom direction of a second absorbent article row accommodated below the first absorbent article row, and at least the first easy-to-tear portion at least partially overlaps the inside visible region.

According to the aspect of the above Clause 7, in a form in which two or more rows of absorbent articles each including a plurality of absorbent articles are accommodated in a top-bottom direction in one packaging bag, the absorbent articles of each example can be easily taken out. For example, by tearing the packaging bag into an upper section and a lower section at the first easy-to-tear portion, the upper portion of the absorbent article of the upper first absorbent article row is exposed, and therefore, the absorbent articles of the first absorbent article row can be easily taken out. After the absorbent articles of the first absorbent article row are finished, the packaging bag can be torn into an upper section and a lower section at the second easy-to-tear portion, which is located lower than the first easy-to-tear portion, whereby the upper portions of the absorbent articles of the lower second absorbent article row are now exposed, and the absorbent articles of the second absorbent article row can be easily taken out. Since at least the first easy-to-tear portion at least partially overlaps the inside visible region, the first easy-to-tear portion that is torn first when the packaging bag is opened can be easily found, and labor or time for opening can be saved.

### (Clause 8)

In the aspect according to Clause 8, information of the absorbent articles is added above the first easy-to-tear portion.

In the aspect according to the above Clause 8, the position at which the information of the absorbent articles is added is above the first easy-to-tear portion that is torn first to open the bag. Therefore, when the packaging bag is torn at the first easy-to-tear portion and the upper portion is removed, the information of the absorbent articles indicated in the remaining portion of the packaging bag can be eliminated or reduced. Therefore, as in the aspect according to Clause 6, this makes it difficult to immediately recognize that the accommodated article is an absorbent article, even in the case where the packaging bag opened by tearing at the first easy-to-tear portion is placed at a conspicuous place.

### (Clause 9)

An aspect according to Clause 9 is an absorbent article packaged product in which a plurality of absorbent articles are accommodated in the absorbent article packaging bag according to any of the aspects according to Clauses 1 to 8.

According to the aspect of the above Clause 9, it is possible to provide an absorbent article packaged product that achieves the same effect as the effect according to any of the aspects of the Clauses 1 to 8.

This application claims priority based on Japanese Patent Application No. 2022-146676 filed on September 15, 2022, the entire contents of which are incorporated herein by reference.

### REFERENCE SIGNS LIST

1 Absorbent article (incontinence pad)
1a, 1b Absorbent article row
10, 10A, 10B, 10C, 10D, 10E, 210, 210A, 210B, 210C Absorbent article packaging bag (packaging bag)
10a Upper section
10b, 10b' Lower section
11, 211 Top surface section
12, 212 Bottom surface portion
15, 215 Surrounding surface portion
15F, 215F Front surface portion
15B, 215B Rear surface portion
15S, 215S Side surface portion
18 Joined portion
18a To-be-joined portion
20, 21, 22 Easy-to-tear portion
30, 31, 32 Inside visible region
30L' Lower inside visible region
30U Upper inside visible region
50 Article-related information
100, 100', 100A, 100B, 100C, 100D, 100E, 200, 200A, 200B, 200C Absorbent article packaged product
210a Upper section
210b Middle section
210c Lower section

## Claims

1. An absorbent article packaging bag that is colored and capable of accommodating a plurality of absorbent articles in an upright state, the absorbent article packaging bag comprising:
a substantially rectangular parallelepiped shape having a top surface portion, a bottom surface portion, and a surrounding surface portion connecting the top surface portion and the bottom surface portion, wherein
the surrounding surface portion has an inside visible region, and part of the absorbent articles is visible through the inside visible region,
an easy-to-tear portion is formed in the surrounding surface portion and with which the surrounding surface portion can be torn in a top-bottom direction, and
the easy-to-tear portion at least partially overlaps the inside visible region.

2. The absorbent article packaging bag according to claim 1, wherein
the easy-to-tear portion is formed in a linear shape, and
a ratio of a portion of the easy-to-tear portion overlapping the inside visible region to an entire length of the easy-to-tear portion is 50% or more.

3. The absorbent article packaging bag according to claim 2, wherein
the easy-to-tear portion is formed in an annular shape in a direction in which the easy-to-tear portion surrounds the absorbent article packaging bag, and
an entirety of the easy-to-tear portion is formed within the inside visible region.

4. The absorbent article packaging bag according to claim 1, wherein
the inside visible region is elongated in an orthogonal direction orthogonal to the top-bottom direction and has a shape that is wider at a center in the orthogonal direction.

5. The absorbent article packaging bag according to claim 1, wherein
the inside visible region includes an upper inside visible region above the easy-to-tear portion and a lower inside visible region below the easy-to-tear portion, and
an area of the lower inside visible region is larger than an area of the upper inside visible region.

6. The absorbent article packaging bag according to claim 1, wherein
information of the absorbent articles is added above the easy-to-tear portion.

7. The absorbent article packaging bag according to claim 1, wherein
two or more absorbent article rows each formed by arranging a plurality of absorbent articles in an upright state can be accommodated in the top-bottom direction,
the easy-to-tear portion includes a first easy-to-tear portion formed within a range in the top-bottom direction of a first absorbent article row that is the uppermost of the two or more absorbent article rows, and a second easy-to-tear portion formed within a range in the top-bottom direction of a second absorbent article row that is accommodated below the first absorbent article row, and
at least the first easy-to-tear portion at least partially overlaps the inside visible region.

8. The absorbent article packaging bag according to claim 7, wherein
the information of the absorbent articles is added above the first easy-to-tear portion.

9. An absorbent article packaged product, comprising:
the absorbent article packaging bag according to any one of claims 1 to 8; and
a plurality of absorbent articles accommodated in the absorbent article packaging bag.
